# EUROPEAN PATENT SPECIFICATION

(11) **EP 1 492 748 B1**
(45) Date of publication and mention of the grant of the patent: **19.05.2010**
(21) Application number: 03719655.7
(22) Date of filing: 09.04.2003
(51) Int. Cl.: C07C 7/04, C07C 7/00, C07C 7/09, F25J 3/06

(54) **OLEFIN PLANT REFRIGERATION SYSTEM**
KÜHLANLAGE IN EINER OLEFIN-ANLAGE
SYSTEME DE REFROIDISSEMENT DANS UNE INSTALLATION DE PRODUCTION D'OLEFINES

(30) Priority: 11.04.2002 US 121151
(43) Date of publication of application: 05.01.2005
(73) Proprietor: Lummus Technology Inc., Bloomfield NJ 07003-3096 (US)
(72) Inventor: WEI, Vitus Tuan, Houston, TX 77063 (US); MA, Qi, Katy, TX 77450 (US); WU, James, Tzong-Chaur, Houston, TX 77083 (US)
(74) Representative: Serjeants
(86) International application number: PCT/US2003/010873
(87) International publication number: WO 2003/087022

(56) References cited:
- EP-A- 0 667 326
- EP-A- 0 667 327
- US-A- 4 456 460
- US-A- 4 584 006
- US-A- 5 377 490
- US-A- 5 379 597
- US-A- 5 626 034
- US-A- 5 979 177
- US-A1- 2002 174 679

## Description

### Background of the Invention

The present invention pertains to a refrigeration system to provide the cooling requirements of an olefin plant. More particularly, the invention is directed to the use of a tertiary or trinary refrigerant comprising a mixture of methane, ethylene and propylene for cooling in an ethylene plant.

Ethylene plants require refrigeration to separate out desired products from the cracking heater effluent. Typically, a propylene and an ethylene refrigerant are used. Often, particularly in systems using low pressure demethanizers where lower temperatures are required, a separate methane refrigeration system is also employed. Thus three separate refrigeration systems are required, cascading from lowest temperature to highest. Three compressor and driver systems complete with suction drums, separate exchangers, piping, etc. are required. An additional methane refrigeration compressor, either reciprocating or centrifugal, can partially offset the capital cost savings resulting from the use of low pressure demethanizers.

Mixed refrigerant systems have been well known in the industry for many decades. In these systems, multiple refrigerants are utilized in a single refrigeration system to provide refrigeration covering a wider range of temperatures, enabling one mixed refrigeration system to replace multiple pure component cascade refrigeration systems. These mixed refrigeration systems have found widespread use in base load liquid natural gas plants. The application of a binary mixed refrigeration system to ethylene plant design is disclosed in U.S. Patent 5,979,177 in which the refrigerant is a mixture of methane and either ethylene or ethane. However, such a binary refrigeration system cascades against a separate propylene refrigeration system to provide the refrigeration in the temperature range of -40°C and warmer. Therefore, two separate refrigeration systems are required.

U.S. Patent Application 2002/0174679 discloses a refrigeration system for an ethylene plant that utilizes a tertiary refrigerant containing methane, ethylene and propylene. The compressor 64 has an inlet line 92 and an effluent line 66. A portion of the refrigerant from the compressor is separated into a methane-rich vapour fraction and a propylene-rich liquid portion. There is nothing to suggest the withdrawal of tertiary refrigerant vapour from an intermediate stage of a multi-stage compressor.

U.S. Patent 5377490 discloses an open-loop mixed refrigerant cycle that utilises components present in the ethylene-containing feed gas as the mixed refrigerant. Refrigeration is provided in a cycle comprising cooling in an ethylene-containing mixed gas stream, sub-cooling the feed condensate with indirect heat exchange, flashing a portion of the sub-cooled condensate for use as a refrigerant for at least one demethanizer column, and flashing a second portion of the sub-cooled condensate for use as a refrigerant to cool the ethylene-containing mixed gas stream. Compressor 117 has inlet lines 29 and 31, and an outlet line 32. Withdrawing tertiary refrigerant vapour from an intermediate stage of a multi-stage compressor is not mentioned.

EP 06673727 discloses an open-loop mixed refrigerant cycle for ethylene recovery. Refrigeration is provided with a cycle utilizing components present in the ethylene-containing feed gas as the mixed refrigerant. The compressor 117 has inlet lines 29 and 31, and an outlet line 31 as shown in D2. Withdrawing tertiary refrigerant vapour from an intermediate stage of a multi-stage compressor is not mentioned.

U.S. Patent 5626034 discloses a process involving rectification zone separation of ethylene-containing gas chilled by two closed mixed refrigerant loops and at least part of a dephlegmated overhead gas. One of the mixed refrigerant loops cools the upper section of the rectification zone and the other cools the lower section. Each loop has its own compressor.

U.S. Patent 5379597 and EP 0667326 disclose closed-loop mixed refrigeration cycles that do not include the withdrawal of vapour from an intermediate stage of a multi-stage compressor.

U.S. Patent 4584006 discloses a process for recovering propane and heavier hydrocarbon components from ethane, methane and lighter constituents of natural gas streams. The system utilises only two stages of compression.

U.S. Patent 4456460 discloses a process for receiving ethane, propane and heavier hydrocarbons from a natural gas stream. The system has a refrigeration cycle with only two stages of compression.

### Summary of the Invention

It is an object of the present invention, therefore, to provide a simplified, single refrigeration system for an olefin plant, particularly an ethylene plant having a low pressure demethanizer, utilizing a mixture of methane, ethylene and propylene as a tertiary refrigerant. This tertiary system replaces the separate propylene, ethylene and methane refrigeration systems associated with a recovery process using a low pressure methanizer. The invention involves the separation of the tertiary refrigerant from a compressor interstage discharge and the final compressor discharge into a methane-rich vapor fraction and two levels of propane-rich liquids so as to provide various temperatures and levels of refrigeration in various heat exchange stages while maintaining a nearly constant refrigerant composition flowing back to the compressor and with the bulk of the total return refrigerant flow going to the first stage compressor suction. This enables the tertiary refrigerant system to compete favourably on a thermodynamic basis with the use of separate compressors for separate refrigerants. This tertiary system can also be applied to an ethylene plant with a high pressure demethanizer in which case the tertiary system only supplies propylene and ethylene refrigeration temperature levels. The objects, arrangement and advantages of the refrigeration system of the present system will be apparent from the description which follows.

The present invention provides a method for cooling charge gas and additional olefin plant process streams as defined in claim 1. Preferred features of that method are defined in the dependent claims.

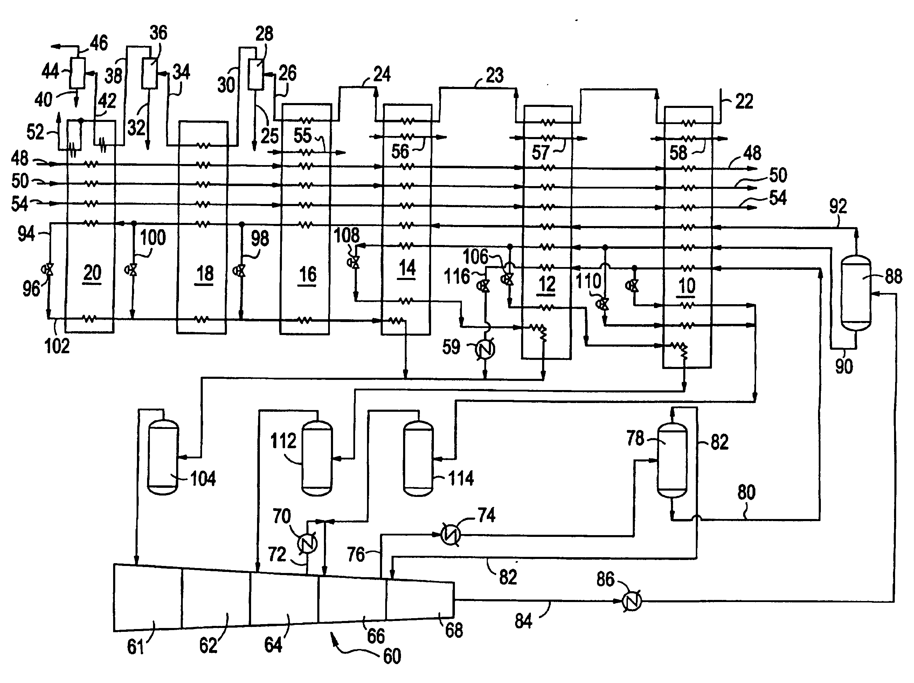

### Brief Description of the Drawing

The drawing is a schematic flow diagram of a portion of an ethylene plant illustrating one embodiment of the refrigeration system of the present invention.

### Description of the Preferred Embodiments

The present invention relates to an olefin plant wherein a pyrolysis gas is first processed to remove methane and hydrogen and then processed in a known manner to produce and separate ethylene as well as propylene and some other by-products. The process will be described in connection with a plant which is primarily for the production of ethylene. The separation of the gases in an ethylene plant through condensation and fractionation at cryogenic temperatures requires refrigeration over a wide temperature range. The capital cost involved in the refrigeration system of an ethylene plant can be a significant part of the overall plant cost. Therefore, capital savings for the refrigeration system will significantly affect the overall plant cost.

Ethylene plants with high pressure demethanizers operate at pressures higher than 2.76 MPa (400 psi) with an overhead temperature typically in the range of -85°C to -100°C. Ethylene refrigeration at approximately -100 to 102°C is typically used to chill and produce overhead reflux. An ethylene plant designed with a low pressure demethanizer which operates below 2.41 MPa (350 psi) and generally in the range of 0.345 to 1.034 MPa (50 to 150 psi) and with overhead temperatures in the range of -110 to -140°C requires methane temperature levels of refrigeration to generate reflux. The advantage of the low pressure demethanizer is the lower total plant power requirement and the lower total plant capital cost while the disadvantage is the lower refrigeration temperature required and, therefore, the need for a methane refrigeration system in addition to the ethylene and propylene refrigeration systems.

The tertiary refrigerant of the present invention comprises a mixture of methane, ethylene and propylene. The percentage of these components can vary depending on the ethylene plant cracking feedstock, the cracking severity and the chilling train pressure among other considerations, but will generally be in the range of 7 to 20 percent methane, 7 to 30 percent ethylene and 50 to 85 percent propylene. A typical composition for an ethylene plant with a low pressure demethanizer would be 10% methane, 10% ethylene and 80% propylene. The use of the tertiary refrigerant provides all the refrigeration loads and temperatures required for an ethylene plant while obviating the need for two or three separate refrigerant systems.

The purpose of the present invention is to provide the necessary refrigeration to separate the hydrogen and methane from the charge gas and provide the feed for the demethanizer as well as provide for the other refrigeration requirements of the entire plant. Referring to the specific embodiment of the invention shown in the drawing which is for a low pressure demethanizer, the tertiary refrigeration system is arranged to provide all of the required levels of refrigeration for an ethylene plant in the series of heat exchangers 10, 12, 14, 16, 18 and 20. These heat exchangers can be combined as fewer units or expanded into a greater number of units depending on the particular needs for any particular ethylene process and in particular on the specific charge gas composition. They are typically plate fin type heat exchangers and are preferably packed inside of a heavily insulated structure referred to as a cold box to prevent heat gain and to localize the low temperature operation. Before describing the tertiary refrigeration system, the flow of the charge gas through the system will be described with examples of specific temperatures for purposes of illustration only.

The charge gas feed 22, which is the pyrolysis gas conditioned as required and cooled, is typically at a temperature of 15 to 20°C and a pressure of 3.45 MPa (500 psi), and is typically a vapor stream. The charge gas contains hydrogen, methane, and C₂ and heavier components including ethylene and propylene. The charge gas 22 is progressively cooled by the refrigeration system of the present invention in the heat exchangers 10, 12, 14, 16, 18 and 20 with appropriate separations being made to produce demethanizer feeds. The charge gas 22 is first cooled in the heat exchangers 10 and 12 down to about -35°C at 23. In heat exchanger 14, the charge gas is cooled from -35°C to -60°C at 23. In heat exchanger 16, it is cooled from -60°C to -72°C with the condensate 25 in the effluent 26 being separated at 28. The condensate 25 is a lower feed to the demethanizer (not shown). The remaining vapor 30 is then cooled from -72°C to -98°C in heat exchanger 18 with the condensate 32 in the effluent 34 being separated at 36. This condensate 32 is a middle feed to the demethanizer. The vapor 38 is then further cooled in heat exchanger 20 from -98°C to -130°C with the condensate 40 in the effluent 42 being separated at 44. The condensate 40 is a top feed to the demethanizer. The remaining vapor 46 is then separated (not shown) to produce the hydrogen stream 48 and the low pressure methane stream 50. The cooling loop 52 is for cooling and partially condensing the low pressure demethanizer overhead to generate reflux. The remaining overhead vapor from the demethanizer forms the high pressure methane stream 54. The hydrogen stream 48 and the low and high pressure methane streams 50 and 54 provide additional cooling in the heat exchangers. To complete the description of the charge gas flow, it is the demethanizer bottoms which contains the C₂ and heavier components which is sent for the recovery of the ethylene and propylene and other components.

In addition to the charge gas stream and the tertiary refrigerant streams, the streams 55, 56, 57 and 58 are various ethylene plant streams at various temperatures which also pass through the heat exchangers for recuperation of cold. Merely as examples, stream 55 is for the recuperation of the cold from the low pressure demethanizer side reboiler. Stream 56 recuperates the cold from the deethanizer feed and the low pressure demethanizer bottom reboiler. Stream 57 is for recuperation of the deethanizer feed, the ethane recycle, the ethylene fractionator side reboiler and bottom reboiler and the ethylene product. The last stream 58 covers the recuperation of cold from the lower deethanizer feed, the ethylene product, the ethane recycle and the refrigeration consumed in a double-pressure depropanizer system.

The maximum efficiency of heat transfer between a warm fluid and a cold fluid is achieved when the temperature difference is low. A mixed refrigerant, such as proposed in this invention, has an increasing temperature with increasing vaporization, at a fixed pressure. This is as distinguished from a pure component refrigerant which vaporizes at a constant temperature at a fixed pressure. Pure component refrigeration systems therefore tend to be more efficient when the process condensing temperatures are unchanged, or relatively unchanged, when being cooled, and relatively less efficient when process temperatures decrease when being cooled. For mixed refrigeration systems, such as proposed in this invention, the relative advantages are reversed.

In an ethylene plant, some of the cooling services requiring refrigeration are at relatively constant temperatures and some are at decreasing temperatures. In the pending U.S. Patent Application Serial No. 09/862,253, entitled, Tertiary Refrigeration System for Ethylene Plants, and filed May 22, 2001, a mixed refrigerant system for ethylene plants is described which emphasizes a constant composition throughout the system. Thus, a somewhat lower efficiency in the constant temperature heat transfer services has been understood. The present invention proposes to improve the efficiency of the mixed refrigeration system by varying the composition of the mixed refrigerant used for these constant temperature heat transfer services. This invention is especially directed to the refrigeration system utilized in the separation of ethylene from ethane which has a very large refrigeration requirement. The concept can also be utilized for other constant temperature heat transfer services with lower heat transfer duty such as the deethanizer.

For the purposes of the present invention, the total duty of the ethylene fractionator condenser 59 is handled outside the coldbox with special consideration. Shell and tube exchangers are typicallly used for the ethylene fractionator condenser heat transfer service although platefin exchangers, as in the cold box, can also be utilized. As known from the thermodynamics, the condensation of the process stream with constant temperature, such as the ethylene fractionator overhead and the deethanizer overhead, as well as the depropanizer overhead if a single low pressure tower is employed, will be less efficient if a mixed refrigeration system is used where the vaporization curve is sloped with temperature. The wide cold-end temperature approach indicates inefficiency and results in higher power consumption for the tertiary refrigeration system. For the deethanizer condenser, the refrigeration can be supplied by the ethylene fractionator side reboiler with near constant temperature on both sides. However, there is no alternative for the ethylene fractionator condenser which is the biggest refrigeration consumer in the ethylene plant. To make the tertiary system competitive in power consumption to a system designed with separate compressors, a concept to generate a heavy refrigerant stream approaching the conventional propylene refrigeration is called for in the tertiary system of the present invention.

Turning now to the refrigeration system per se, the tertiary refrigerant as identified earlier is a mixture of methane, ethylene and propylene and is compressed by the multistage refrigeration compressor 60. In the illustrated embodiment, there are five compressor stages 61, 62, 64, 66 and 68 with two interstage coolers. The interstage cooler 70 is at the third stage discharge 72 while the interstage cooler 74 is at the fourth stage discharge 76. The liquid in this fourth stage discharge after cooling is separated in the drum 78 to provide the heavy refrigerant 80. The remaining vapor 82 from drum 78 is returned to the fifth compressor stage 68 and extracted as the fifth stage final effluent 84. This final effluent 84 is cooled and partially condensed at 86 and then separated in drum 88 to generate a medium refrigerant 90 and a light refrigerant 92 by phase separation. The typical operating conditions and the range of operating conditions for the compressor are as follows:

| | **Range of Suction Pressure** | **Typical Suction Conditions** | |
|---|---|---|---|
| | Mpa | Mpa | Degree C |
| 1^{st} Stage | 0.011 - 0.016 | 0.014 | -40 |
| 2^{nd} Stage | 0.4-0.55 | 0.46 | 9.0 |
| 3^{rd} Stage | 0.7-0.95 | 0.86 | 47 |
| 4^{th} Stage | 1.1 - 2.0 | 1.5 | 37 |
| 5^{th} Stage | 2.8-3.2 | 3.0 | 45 |

The light refrigerant 92 from the drum 88 passes through all of the heat exchangers 10 to 20 and is condensed and subcooled in the process. It is subcooled to -130°C at the exit 94 from heat exchanger 20 and then flashed through valve 96 to provide the lowest refrigeration temperature of -140°C to -145°C. This level of refrigeration provides the cooling of the charge gas stream at 42 down to -130°C or lower and to provide sufficient cooling in the loop 52 to generate reflux from the demethanizer overhead.

The charge gas temperature in streams 26 and 34 are typically controlled at -72°C and -98 °C respectively by controlling the flow of the light refrigerant in streams 98 and 100. Typically, the refrigeration supplied by the stream 102 will meet the refrigeration demand in heat exchangers 20, 18 and 16. The light refrigerant is finally superheated to -45°C in heat exchanger 14. This provides the desired superheat temperature of 5 to 15°C when it is mixed with portions of the heavy and medium refrigerate streams for return to the first stage suction drum 104.

The liquid 90 from the drum 88 is the medium refrigerant which is subcooled as it passes through heat exchangers 10, 12 and 14. This medium refrigerant controls the temperature of the charge gas at 23 and 24 by flashing the subcooled refrigerant through valves 106 and 108. From valve 108, the medium refrigerate flows back through heat exchangers 14 and 12 and then to the suction drum 104 for the first stage 61 of the compressor. From valve 106, the medium refrigerant flows back through heat exchangers 12 and 10 and then to the suction drum 112 for the third stage 64 of the compressor. The liquid level in drum 88 is controlled by adjusting the valve 110 and providing limited refrigeration to heat exchanger 10. This portion of the medium is then fed to the suction drum 114 for the fourth stage 66 of the compressor.

The heavy refrigerant 80 from the drum 78 is about 88% propylene. This liquid supplies two major duties, i.e., the cooling for the ethylene condenser 59 and the major refrigeration demand in heat exchanger 10 to support the self-refrigeration of the tertiary refrigeration system. The degree of subcooling of the heavy refrigerant exiting the heat exchanger 12 at 116 is flexible between -10°C and -35°C. The following table is a summary of the suction streams to the compressor and the compressor flows.

| **Stages** | **Type of Refrigerant** | **Wt% of total flow** | **Ave. MW** |
|---|---|---|---|
| 1^{st} Stage Suction | 100% Light Refrigerant | 9.0 | |
| | Medium Refrigerant | 3.5 | |
| | Heavy Refrigerant | 56.0 | |
| 1^{st} & 2^{nd} Stage Flow | | 68.5 | 38.14 |
| 3^{rd} Stage Side Inlet | Medium Refrigerant | 3.0 | |
| 3^{rd} Stage Flow | | 71.5 | 38.14 |
| 4^{th} Stage Side Inlet | Medium Refrigerant | 7.0 | |
| | Heavy Refrigerant | 21.5 | |
| 4^{th} Stage Flow | | 100 | 38.48 |
| 5^{th} Stage Suction and Discharge Flow | Light & Medium Refrigerant | 22.5 | 34.35 |

As shown by the above table, the split of the refrigerant for the purpose of energy saving and then the recombination of the refrigerants, particularly the recombination in the first compressor stage of the light and most of the heavy refrigerants along with some medium refrigerant to provide almost 70% of the total flow in the first stage stabilizes the compressor wheels. With 70% of the total flow in the first stage and a relatively uniform molecular weight throughout, a normal speed control of the turbine by the first stage suction drum pressure becomes equally applicable to the tertiary refrigerant compressor system as to a single refrigerant compressor system. After the extraction of the heavy refrigerant from the fourth stage flow, the flow and the molecular weight in the fifth stage becomes substantially lower. However, the fifth stage compression can be designed and the loading variations can be controlled by the recycle flow to the first stage to minimize the effects. With respect to the control of the process chilling duties, the variables which can be used include the control of the critical temperature, the adjustment of the overall refrigerant composition, the adjustment of the temperatures in the separation drums 78 and 88 and the adjustment of the compressor operating conditions.

The closed loop tertiary refrigeration system with one or more side draws from the compressor inter-stages of the present invention provides a versatile system in which various refrigerant compositions can be formed and various refrigeration levels can be provided. This provides precise temperature control in an efficient and economical manner. Therefore, a single closed loop tertiary refrigeration system can adequately provide all the necessary refrigeration to the entire ethylene plant with either a low pressure or high pressure demethanizer at a competitive power consumption and a lower overall plant cost.

## Claims

1. In a process for the production of olefins from a charge gas containing hydrogen, methane, ethylene and other C₂ and heavier hydrocarbons wherein said charge gas and additional olefin plant process streams are cooled by a refrigeration system having a series of heat exchangers, a method for cooling said charge gas and additional olefin plant process streams by the use of a tertiary refrigerant in said refrigeration system comprising the steps of:
(a) compressing a tertiary refrigerant comprising a selected mixture consisting of methane, ethylene and propylene in a multistage compressor having a first stage and a last stage and at least one intermediate stage;
(b) withdrawing at least a portion of said tertiary refrigerant vapor from one of said intermediate stages;
(c) cooling said withdrawn portion of said tertiary refrigerant vapor to form a remaining tertiary refrigerant vapor and a heavy liquid refrigerant having a greater percentage of propylene than said selected mixture;
(d) separating said heavy liquid refrigerant from said remaining tertiary refrigerant vapor, and returning said remaining tertiary refrigerant vapor to and through said last stage;
(e) extracting said remaining tertiary refrigerant vapor from said last stage of said compressor and cooling to condense a portion thereof thereby forming a medium liquid refrigerant and forming a light vapor refrigerant of the uncondensed portion thereof;
(f) bringing said heavy and medium liquid refrigerants and said light vapor refrigerant Into heat exchange contact with themselves and each other and with said charge gas and additional olefin plant process streams in said series of heat exchangers whereby said charge gas and additional olefin plant process streams are cooled and said heavy and medium liquid refrigerants are heated and vaporized and said light vapor refrigerant is first cooled and at least partially condensed and then vaporized; and
(g) returning said light and medium and heavy vaporized refrigerants to said compressor.

2. In a process as recited in claim 1 wherein said step of withdrawing at least a portion of said tertiary refrigerant vapor from one of said intermediate stages comprises withdrawing all of said tertiary refrigerant vapor from said stage.

3. In a process as recited in claim 2 wherein said one of said intermediate stages is the penultimate stage.

4. In a process as recited in claim 1 wherein said heavy liquid refrigerant in step (c) has a propylene content of about 88%.

## Patentansprüche

1. In einem Verfahren für die Herstellung von Olefinen aus einer Gascharge, die Wasserstoff, Methan, Ethylen und andere C₂ und schwerere Kohlenwasserstoffe enthält, wobei die genannte Gascharge und zusätzliche Olefinanlagen-Prozessströme durch ein Kühlsystem, das eine Reihe von Wärmeaustauschern aufweist, gekühlt werden, ein Verfahren für das Kühlen der genannten Gascharge und der genannten zusätzlichen Olefinanlagen-Prozessströme durch die Verwendung eines tertiären Kühlmittels in dem genannten Kühlsystem, das die Schritte umfasst:
(a) Komprimieren eines tertiären Kühlmittels, das eine ausgewählte Mischung umfasst, die aus Methan, Ethylen und Propylen besteht, in einem mehrstufigen Kompressor, der eine erste Stufe und eine letzte Stufe und mindestens eine Zwischenstufe aufweist;
(b) Abziehen mindestens eines Teils des genannten tertiären Kühlmitteldampfes von einer der genannten Zwischenstufen;
(c) Kühlen des genannten abgezogenen Teils des genannten tertiären Kühlmitteldampfes um einen verbleibende tertiären Kühlmitteldampf und ein schweres Flüssigkühlmittel, das eine höhere Prozentzahl an Propylen aufweist als die genannte ausgewählte Mischung, zu bilden;
(d) Abtrennen des genannten schweren Flüssigkühlmittel von dem verbleibenden tertiären Kühlmitteldampf und Zurückführen des genannten verbleibenden tertiären Kühlmitteldampfes zu und durch die genannte letzte Stufe;
(e) Extrahieren des genannten verbleibenden tertiären Kühlmitteldampfes aus der genannte letzten Stufe des genannte Kompressors und Kühlen, um einen Teil davon zu kondensieren, dabei Bilden eines mittleren Flüssigkühlmittels und Bilden eines leichten Dampfkühlmittels aus dem unkondensierten Teil davon;
(f) Bringen der genannten schweren und mittleren Flüssigkühlmittel und des genannten leichten Dampfkühlmittels in Wärmeaustauschkontakt mit sich selbst und miteinander und mit der genannten Gascharge und den genannten zusätzlichen Olefinanlagen-Prozessströmen in der genannte Reihe von Wärmeaustauschern, wobei das genannte Chargengas und die genannten zusätzlichen Olefinanlagen-Prozessströme gekühlt werden und die genannten schweren und mittleren Flüssigkühlmitte erhitzt und verdampft werden und das genannten leichte Dampfkühlmittel zuerst abgekühlt und mindestens partiell kondensiert und dann verdampft wird; und
(g) Zurückführen der genannten leichten und mittleren und schweren verdampften Kühlmittel zu dem genannten Kompressor.

2. In einem Verfahren wie rezitiert in Anspruch 1, wobei der genannte Schritt des Abziehens mindestens eines Teils des genannten tertiären Kühlmitteldampfes aus einer der genannten Zwischenstufen das Abziehen des gesamten genannten tertiären Kühlmitteldampfes aus der genannten Stufe umfasst.

3. In einem Verfahren wie rezitiert in Anspruch 2, wobei die genannte Eine der genannten Zwischenstufen die vorletzte Stufe ist.

4. In einem Verfahren wie rezitiert in Anspruch 1, wobei das genannte schwere Flüssigkühlmittel in Schritt (c) einen Propylenanteil von etwa 88% hat.

## Revendications

1. Dans un processus de production d'oléfines à partir d'un gaz de charge contenant de l'hydrogène, du méthane, de l'éthylène et autre C₂ et des hydrocarbures lourds, dans lequel ledit gaz de charge et des flux de traitement d'installation d'oléfine additionnels sont refroidis par un système de réfrigération comportant une série d'échangeurs de chaleur, un procédé pour refroidir ledit gaz de charge et les flux de traitement d'installation d'oléfine additionnels à l'aide d'un fluide frigorigène tertiaire dans ledit système de réfrigération, comprenant les étapes consistant à :
(a) comprimer un fluide frigorigène tertiaire comprenant un mélange sélectionné constitué de méthane, d'éthylène et de propylène dans un compresseur à étages multiples comprenant un premier étage et un dernier étage et au moins un étage intermédiaire ;
(b) retirer au moins une partie de la vapeur dudit fluide frigorigène tertiaire de l'un desdits étages intermédiaires ;
(c) refroidir ladite partie retirée de vapeur dudit fluide frigorigène tertiaire pour former une vapeur restante de fluide frigorigène tertiaire et un fluide frigorigène liquide lourd ayant un pourcentage de propylène plus grand que ledit mélange sélectionné ;
(d) séparer ledit fluide frigorigène liquide lourd de ladite vapeur restante de fluide frigorigène tertiaire, et renvoyer ladite vapeur restante de fluide frigorigène tertiaire vers et à travers ledit dernier étage ;
(e) extraire ladite vapeur restante de fluide frigorigène tertiaire dudit dernier étage dudit compresseur et refroidir pour condenser une partie de celle-ci, pour ainsi former un fluide frigorigène liquide moyen et former un fluide frigorigène en vapeur légère de la partie non condensée de celle-ci ;
(f) mettre en contact lesdits fluides frigorigènes liquides lourd et moyen et ledit fluide frigorigène à vapeur légère en contact d'échange de chaleur avec eux-mêmes et entre eux et avec ledit gaz de charge et les flux de traitement d'installation d'oléfine additionnels dans ladite série d'échangeurs de chaleur, moyennant quoi ledit gaz de charge et les flux de traitement d'installation d'oléfine additionnels sont refroidis et lesdits fluides frigorigènes liquides lourd et moyen sont chauffés et vaporisés et ledit fluide frigorigène à vapeur légère est d'abord refroidi, puis au moins partiellement condensé, puis vaporisé ; et
(g) renvoyer lesdits fluides frigorigènes vaporisés léger, moyen et lourd vers ledit compresseur.

2. Dans un processus selon la revendication 1, ladite étape de retrait d'au moins une partie de la vapeur dudit fluide frigorigène tertiaire dé l'un desdits étages intermédiaires comprend le retrait de la totalité de la vapeur dudit fluide frigorigène tertiaire dudit étage.

3. Dans un processus selon la revendication 2, ledit étage desdits étages intermédiaires est l'avant-dernier étage.

4. Dans un processus selon la revendication 1, ledit fluide frigorigène liquide lourd à l'étape (c) présente une teneur en propylène d'environ 88 %.
